# EUROPÄISCHE PATENTSCHRIFT

(11) **EP 1 157 676 B1**
(45) Veröffentlichungstag und Bekanntmachung des Hinweises auf die Patenterteilung: **03.08.2005**
(21) Anmeldenummer: 01810416.6
(22) Anmeldetag: 27.04.2001
(51) Int. Cl.: A61F 2/44, A61L 27/52

(54) **Bandscheibenersatz für den Kern einer Bandscheibe**
Spinal disc replacement for the core of an intervertebral disc
Disque intervertébral de remplacement pour le noyau d'un disque intervertébral

(30) Priorität: 26.05.2000 EP 00810457
(43) Veröffentlichungstag der Anmeldung: 28.11.2001
(73) Patentinhaber: Zimmer GmbH, 8404 Winterthur (CH)
(72) Erfinder: Studer, Armin, 8400 Winterthur (CH); Schärer, Nicolas, 8472 Seuzach (CH)
(74) Vertreter: Manitz, Finsterwald & Partner Gbr

(56) Entgegenhaltungen:
- EP-A- 0 700 671
- EP-A- 0 773 008
- US-A- 3 867 728

## Beschreibung

Die Erfindung handelt von einem Bandscheibenersatz für den Kern einer Bandscheibe, mit einer flachen Spirale aus Kunststoff, die mit Hilfswerkzeugen länglich ausstreckbar ist und die sich im kräftefreien Zustand selbständig zu ihrer Spiralform aufwickelt.

Beim Einbringen der sich selbst aufrollenden Spirale in eine Kavität füllt die Spirale in radialer Richtung durch das Nachschieben von Spiralenwindungen den Querschnitt vollständig aus. In der Richtung der Achse der Spirale bleibt die Höhe jedoch unverändert und es verbleibt in axialer Richtung nach dem Einsetzen ein zum Einsetzen notwendiges Minimalspiel. Beispiele für einen derartigen Bandscheibenersatz werden in US 3867728 und US 5919235 gegeben

Aufgabe der Erfindung ist es, diesen Zustand zu verbessern. Diese Aufgabe wird dadurch gelöst, dass die Spirale längs ihres aufgewickelten Körpers auf der Ober- oder Unterseite einen oder mehrere Körpern aus einem Hydrogel aufweist, welche durch die Aufnahme von Wasser ihr Volumen vergrössern und über die Oberseite oder Unterseite der Spirale vorstehen.

Eine solche Anordnung hat den Vorteil, dass in axialer Richtung nicht nur ein Anliegen stattfindet, sondern dass in axialer Richtung ein Druck aufgebaut wird. Wenn der oder die Hydrogelkörper nach dem Quellen der Spiralenober- oder -unterseite noch zusätzlich eine von einer Ebene abweichende Struktur geben, trägt dies zur Verankerung der einzelnen Windungen der Spirale bei. Es besteht für die Langzeitbetrachtung ein geringeres Risiko, dass sich die Spirale unter einem Axialdruck radial nach aussen verformt indem Windungen sich gleitend gegeneinander verschieben können.

Vorteilhafte Weiterbildungen der Erfindungen ergeben sich aus den abhängigen Ansprüchen 2 bis 13. So hat die Suche nach geeigneten Kunststoffen für eine sich selbst aufwickelnde Spirale ergeben, dass ein Polycarbonaturethan sich vorzüglich für deren Herstellung eignet, während die Hydrogelkörper aus PVP (Polyvinylpyrrolidon), aus PVA (Polyvinylalcohol), aus PAN (Polyacrylnitril), aus PAM (Polyacryamid) oder aus PHEMA (Polyhydroxyethylmetracrylat) bestehen.

Wenn die Hydrogelkörper als Einzelkörper in Aussparungen oder Durchbrüchen der Spirale eingesetzt sind, beispielsweise in Form von Stiften, kann ihre Höhe grösser als ihr Durchmesser gewählt werden, um hauptsächlich eine Vergrösserung durch Quellen in der Achsrichtung der Spirale zu erreichen. Ein weiterer Vorteil ergibt sich, wenn solche Stifte nicht auf der Mittellinie sondern mehr zur Aussenseite einer Windung positioniert sind. Sobald der Stift eine gewisse Vorspannung erzeugt, gibt die Aussenseite mit der geringeren Wandstärke nach und es entsteht eine zusätzliche Krümmung.

Eine weitere Wirkung lässt sich erzielen, wenn die Hydrogelkörper also zum Beispiel die Stifte im Bereich der Mitte der Spirale weiter als am Rand der Spirale vorstehen, um eine konvexe Hüllfläche über die Stifte zu erzeugen. Der Vorteil einer solchen konvexen Hüllfläche liegt darin, dass sie sich weniger zu ihrer Gegenfläche verschieben kann und auch gewisse Schubkräfte überträgt.

Es kann aber auch ein Hydrogelkörper längs der Spirale auf der Oberseite und/oder der Unterseite verlaufen, um über eine gewisse Anzahl von Windungen eine Volumenvergrösserung in der Richtung der Achse der Spirale zu erzeugen. Dazu besteht eine Rinne längs der Spirale, die hinterschnitten sein kann, um ein passendes Hydrogelband einzulegen. Der eingelegte wurmartige Hydrogelkörper kann dabei aus Einzelkörpern bestehen, die zur Innenseite der Windung durch ein nicht quellendes Band zusammengehalten sind, in Längsrichtung einander jedoch berühren. Ein derartiger in eine Rinne eingepresster Hydrogelkörper wird mit dem Quellen die Krümmung der Spirale ebenfalls unterstützen.

Ein ähnlicher Effekt entsteht auch, wenn ringförmige Hydrogelkörper auf der Spirale aufgereiht sind, die sich im dehydrierten Zustand längs der Spirale nur auf der Aussenseite einer Windung berühren und beim Quellen auf der Aussenseite Druck erzeugen, um die Krümmung der Spirale zu unterstützen.

Eine weitere Möglichkeit besteht darin, bei einem wurmartigen Hydrogelband auf der Innenseite Spickel auszuschneiden und das Band anschliessend in eine Längsrinne der Spirale einzupressen oder einzukleben.

Eine weitere Möglichkeit besteht darin, spiralförmige Hydrogelteile und Spiralen aus Kunststoff in Achsrichtung der Spirale zu schichten und durch Kleben oder Anschmelzen zu einer Sandwichkonstruktion zu verarbeiten, wobei auch hier Spickelausschnitte in den spiralförmigen Hydrogelteilen auf der Innenseite der Spirale ein Strecken der Spirale vor dem Einschieben in eine Kavität erleichtern und mit dem Quellen eine Krümmung der Spirale unterstützen.

Im folgenden wird die Erfindung anhand von Ausführungsbeispielen beschrieben. Es zeigen:
- Fig. 1: Schematisch die Draufsicht auf eine Spirale mit einer Vielzahl von Hydrogelkörpern, die in der Richtung der Spiralachse in der Spirale eingesetzt sind;
- Fig. 2: schematisch einen Schnitt längs der Achse der Spirale von Figur 1;
- Fig. 3: schematisch eine Ansicht einer Ausführung wie in Figur 1;
- Fig. 4: schematisch eine Ansicht analog zu Figur 3 mit spiralförmigen Hydrogelkörpern, die auf der Oberseite und der Unterseite der Spirale befestigt sind;
- Fig. 5: schematisch einen Schnitt durch eine Spiralwindung mit einem Hydrogelstift, der in einem Durchbruch der Spirale eingesteckt ist;
- Fig. 6: schematisch einen Schnitt durch eine Spiralwindung, die im Bereich des Schnitts von einem ringförmigen Hydrogelkörper umgeben ist;
- Fig. 7: schematisch einen Schnitt durch eine Spiralwindung mit auf der Oberseite und Unterseite in Aussparungen eingesetzten Hydrogelstiften;
- Fig. 8,9,10,12 und 13: schematisch einen Schnitt durch eine Spiralwindung mit spiralförmigen Hydrogelkörpern, die in hinterschnittenen Rinnen in der Spirale eingelegt wird;
- Fig. 11, 14 und 16: schematisch einen Schnitt durch eine Spiralwindung, bei welcher spiralförmige Hydrogelteile durch einen Kleber oder durch Anschmelzen mit einer oder zwei Spiralen verbunden sind; und
- Fig. 15: schematisch einen Schnitt durch eine Spiralwindung in Form einer Sandwichkonstruktion aus Kunststoffspiralen und Hydrogelspiralen, die durch Kleben oder Anschmelzen miteinander verbunden sind.

Mit den Figuren ist ein Bandscheibenersatz für den Kern einer Bandscheibe gezeigt, welcher aus einer flachen Spirale 2 aus Kunststoff besteht, die mit Hilfswerkzeugen länglich ausstreckbar ist und die sich im kräftefreien Zustand selbständig zu ihrer Spiralform aufwickelt. Die Spirale 2 ist längs ihres aufgewickelten Körpers 3, 4, 5 auf der Ober- oder Unterseite 18, 19 mit einem oder mehreren Körpern 9, 10, 11 aus einem Hydrogel verbunden, welche durch die Aufnahme von Wasser ihr Volumen vergrössern und über die Oberseite 18 oder Unterseite 19 der Spirale 2 vorstehen.

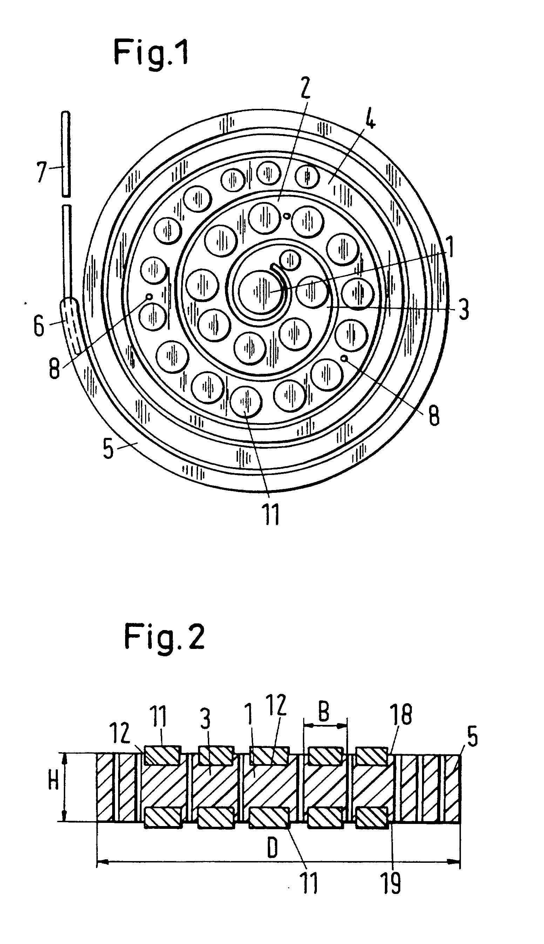

Das Ausführungsbeispiel der Figuren 1, 2, 3 zeigt eine Spirale 2 aus Kunststoff wie sie in der U.S. 5,919,235 beschrieben ist, mit einem Mittelteil 1, an den einige in radialer Richtung dicke Spiralwindungen 3 anschliessen, mit einem Übergang 4, an dem sich die radiale Breite B der Windungen 5 verringert und mit einem Ende 6 der Windungen, an dem eine Versteifung 7 angebracht ist. Die Höhe H und der Durchmesser D der aufgewickelten Spirale 2 entsprechen den Abmessungen eines Bandscheibenkerns. Metallische Marker 8 lassen eine Lagekontrolle mit Durchleuchten zu. Im Mittelteil 1 und in den Spiralwindungen 3 mit grosser radialer Breite B sind Aussparungen 12 auf der Oberseite 18 und auf der Unterseite 19 angebracht, in welche dehydrierte Hydrogelkörper 11 in Form von Stiften eingepresst sind. Die Stifte können im dehydrierten Zustand mit der Oberfläche der Spirale 2 an Ober- und Unterseite 18, 19 abschliessen. Mit der Wasseraufnahme wachsen die Stifte, wobei das Wachstum nach oben oder unten im Vergleich zum radialen Wachstum der Stifte stark davon abhängt, um wieviel die Länge der Stifte grösser als ihr Durchmesser ist. Eine in einer Kavität eingeführte Spirale 2, die auf der Ober- und Unterseite genügend Spiel hat, um sich selbst aufzuwickeln, verkeilt sich zusätzlich auf ihrer Ober- und Unterseite, wenn die eingesetzten Hydrogelstifte im Beisein von Wasser quellen.

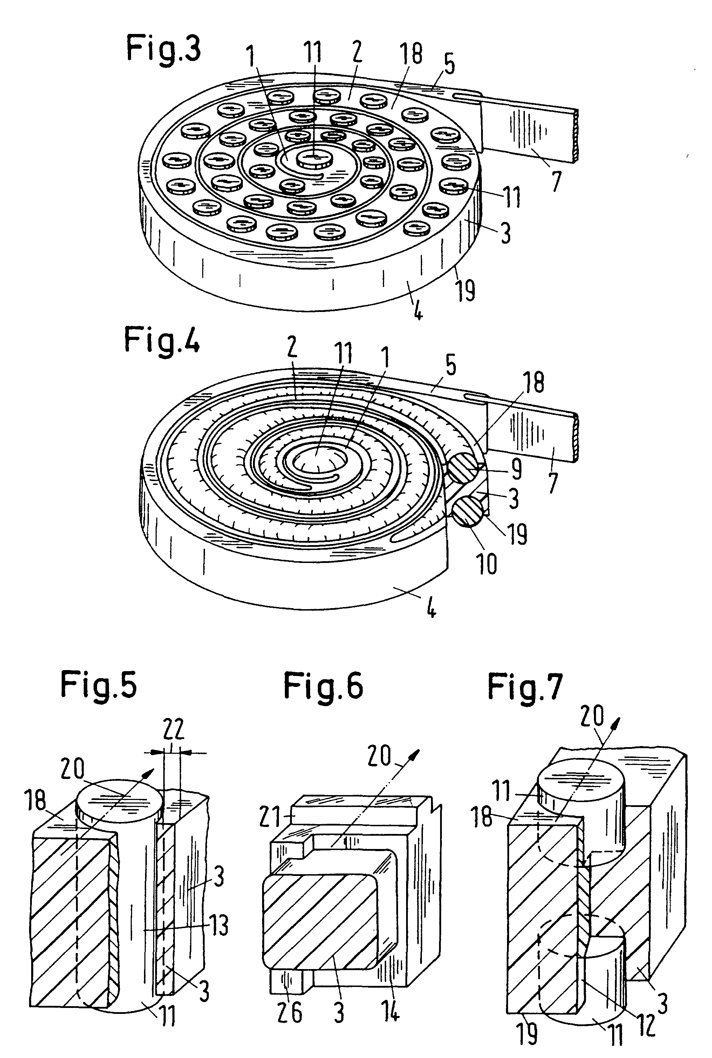

In Figur 7 entspricht die Höhe der in Längsrichtung 20 in der Spiralwindung 3 in Aussparungen 12 verteilten Hydrogelkörper 11 in etwa deren Durchmesser. In Figur 5 sind durchgehende Hydrogelkörper 11 in Längsrichtung 20 in Durchbrüchen 13 verteilt, deren Höhe einem Mehrfachen ihres Durchmessers entspricht. Die Wandstärke 22 auf der Aussenseite der Spiralwindung 3 ist geringer als auf der Innenseite, um mit dem Quellen der Hydrogelkörper 11 eine grössere Längung in der dünneren Seitenwand und eine Unterstützung der Krümmung zu erreichen.

Im Ausführungsbeispiel von Figur 4 sind im Bereich der breiten Spiralwindungen 3 auf der Oberseite 18 und auf der Unterseite 19 wurmartige Hydrogelkörper in Rinnen der Spirale 2 befestigt. Ausserdem sind im Mittelteil 1 separate Hydrogelkörper 11 analog zu Figur 3 eingesetzt. An den Bereich der breiten Spiralwindungen 3 schliesst ein Übergang 4 zu dünneren Spiralwindungen 5 an, an deren Ende eine Versteifung 7 befestigt ist, die das Einschieben der Spirale im gestreckten Zustand erleichtert.

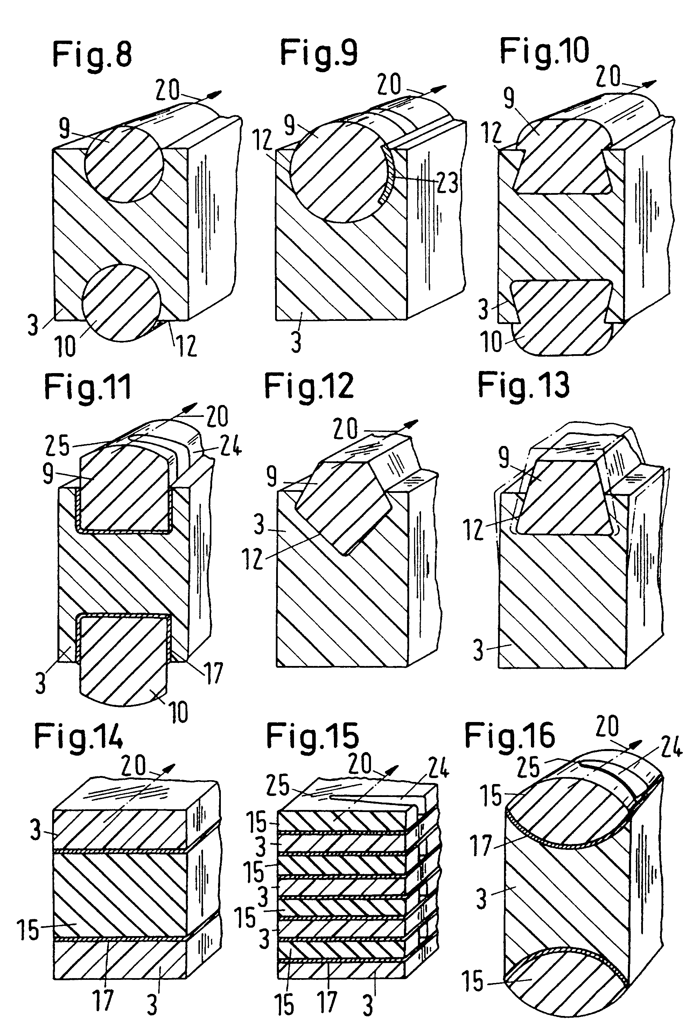

Die Befestigung und die Struktur dieser wurmartigen Hydrogelkörper 9, 10 hängt weitgehend von ihrer Elastizität ab, da die aufgerollte Spirale mit den Hydrogelkörpern 9, 10 für das Einschieben in eine Kavität ausgestreckt werden muss. Ein im dehydrierten Zustand elastisches Hydrogel 9 lässt sich als Spirale ausschneiden und wie in Figur 13 in einer Aussparung 12 in Form einer hinterschnittenen Rinne der Kunststoffspirale 3 einpressen. In Figur 13 sind mit Phantomlinien das Quellen und die Formänderung von Hydrogelkörper 9 und Spiralwindung 3 angedeutet.

Die Figuren 8, 10 und 12 zeigen ebenfalls Hydrogelkörper 9, 10 als durchgehende Spiralen, die in Aussparungen 12 in Form von hinterschnittenen Rinnen eingelegt sind. Die Befestigung kann durch die Hinterschneidung selbst oder durch Kleben oder Anschmelzen erfolgen.

Bei Hydrogelkörpern, die weniger elastisch sind, wird die wurmartige Struktur in Elemente unterteilt, die über schmale und daher eher biegsame Brücken verbunden sind. So zeigt Figur 9 einen in einer Aussparung 12 eingelegten Hydrogelkörper 9, der aus Einzelkörpern besteht, die auf der Innenseite der Spiralwindung 3 mit einem Band 23 verbunden sind, aber einen Abstand zueinander aufweisen, der im dehydrierten Zustand eine Einrollen und ein Strecken der Spirale 2 erlaubt. Wenn der Abstand dieser Einzelkörper auf der Innenseite, wo das Band ist, grösser zueinander ist, dann findet beim Qellen der Einzelkörper der erste Kontakt zueinander auf der Aussenseite statt, was die Krümmung der Spirale 2 ebenfalls unterstützt.

Eine ähnliche Wirkung wird mit einer Anordnung nach den Figuren 11 und 16 erreicht, bei denen wurmartige Hydrogelkörper 9, 10, 15 über einen Kleber 17 oder durch Anschmelzen mit den Spiralwindungen 3 verbunden sind, aber zur Innenseite der Spirale spickelförmige Ausschnitte 24 aufweisen und auf der Aussenseite über eine biegbare Brücke 25 miteinander verbunden sind. Beim Auslängen der Spirale 2 können die Spiralwindungen 3 im Bereich der Ausschnitte 24 nachgeben, während beim Quellen der Hydrogelkörper 9, 10, 15 die Krümmung über den Brücken 25 unterstützt wird.

In Figur 15 ist eine Sandwichkonstruktion von übereinander geschichteten und miteinander verbundenen Spiralenwindungen 3 aus Kunststoff und spiralförmigen Hydrogelteilen 15 gezeigt. Die Hydrogelteile 15 haben ebenfalls spickelförmige Ausschnitte 24 zur Innenseite der Spirale und Brücken 25 an der Aussenseite der Spirale um ein Ausstrecken der Spirale zu erleichtern und beim Quellen der Hydrogelteile die Krümmung der Spirale zu unterstützen. Die Verbindung der einzelnen Lagen erfolgt durch einen Kleber 17 oder durch Anschmelzen. Das Anschmelzen erfolgt durch Erwärmen einer der beiden Oberflächen vor dem Zusammenpressen durch Wärmestrahlung oder durch Heissluft.

In Figur 14 besteht eine Sandwichkonstruktion analog zu Figur 15, wobei zwei Spiralenwindungen 3 ein spiralförmiges Hydrogelteil 15 zwischen sich aufnehmen.

Ein weiteres Beispiel ist in Figur 6 gezeigt, bei dem ringförmige Körper 14 aus Hydrogel - wie Perlen auf einer Schnur - auf einer Spiralwindung 3 aufgereiht sind. Die ringförmigen Hydrogelkörper 14 besitzen auf der Aussenseite der Spirale in Längsrichtung 20 vorstehende Vorsprünge, um die Hydrogelkörper zu distanzieren, und auf der Oberseite Stufen 21, die sich in einer Gegenfläche festsetzen können.

## Patentansprüche

1. Bandscheibenersatz für den Kern einer Bandscheibe, mit einer flachen Spirale (2) aus Kunststoff, die mit Hilfswerkzeugen länglich ausstreckbar ist und die sich im kräftefreien Zustand selbständig zu ihrer Spiralform aufwickelt, **dadurch gekennzeichnet, dass** die Spirale (2) längs ihres aufgewickelten Körpers (3, 4, 5) auf der Ober- oder Unterseite (18, 19) einen oder mehrere Körper (9, 10, 11) aus einem Hydrogel aufweist, welche durch die Aufnahme von Wasser ihr Volumen vergrössern und über die Oberseite (18) oder Unterseite (19) der Spirale (2) vorstehen.

2. Bandscheibenersatz nach Anspruch 1, **dadurch gekennzeichnet, dass** die Spirale (2) aus einem Polycarbonaturethan besteht.

3. Bandscheibenersatz nach einem der Ansprüche 1 oder 2, **dadurch gekennzeichnet, dass** der oder die Hydrogelkörper (9, 10, 11) aus PVP (Polyvinylpyrrolidon), aus PVA (Polyvinylalcohol), aus PAN (Polyacrylnitril), aus PAM (Polyacryamid) oder aus PHEMA (Polyhydroxyethylmetracrylat) bestehen.

4. Bandscheibenersatz nach einem der Ansprüche 1 bis 3, **dadurch gekennzeichnet, dass** mehrere Einzelkörper (11) aus Hydrogel längs der Spirale (2) auf der Oberseite (18) oder auf der Unterseite (19) in Aussparungen (12) der Spirale (2) eingesetzt sind.

5. Bandscheibenersatz nach einem der Ansprüche 1 bis 3, **dadurch gekennzeichnet, dass** Einzelkörper (11) aus Hydrogel längs der Spirale in Durchbrüchen (13) eingesetzt sind.

6. Bandscheibenersatz nach einem der Ansprüche 1 bis 3, **dadurch gekennzeichnet, dass** auf der Oberseite oder auf der Unterseite sich ein Hydrogelkörper (9, 10) längs der Spirale (2) erstreckt und mit dieser verbunden ist.

7. Bandscheibenersatz nach einem der Ansprüche 1 bis 3, **dadurch gekennzeichnet, dass** die Spirale in Längsrichtung (20) über eine vorgegebene Länge von einem oder mehreren ringförmigen Hydrogelkörpern (14) umgeben ist.

8. Bandscheibenersatz nach einem der Ansprüche 1 bis 3, **dadurch gekennzeichnet, dass** mehrere Spiralen (2) aus Kunststoff und mehrere spiralförmige Hydrogelteile (15) in einer Sandwich-Konstruktion (16) verbunden sind.

9. Bandscheibenersatz nach einem der Ansprüche 1 bis 8, **dadurch gekennzeichnet, dass** Spirale und Hydrogelteile durch einen elastischen Kleber (17) verbunden sind.

10. Bandscheibenersatz nach einem der Ansprüche 1 bis 8, **dadurch gekennzeichnet, dass** Spirale (2) und Hydrogelteile (9, 10, 11) durch Anschmelzen miteinander verbunden sind.

11. Bandscheibenersatz nach einem der vorangehenden Ansprüche, **dadurch gekennzeichnet, dass** Windungen (3) der Spirale (2) an ihrer Ober- oder Unterseite (18, 19) in Längsrichtung (20) im gequollenen Zustand der Hydrogelkörper (9, 10, 11) eine Stufung (21) aufweisen.

12. Bandscheibenersatz nach Anspruch 4 oder 5, **dadurch gekennzeichnet, dass** die Einzelkörper (11) in Aussparungen (12) oder Durchbrüchen (13) eingesetzt sind, die eine geringere Wandstärke (22) zur Aussenseite als zur Innenseite der Spirale (2) aufweisen, um den Aufrolleffekt zu verstärken.

13. Bandscheibenersatz nach einem der vorangehenden Ansprüche, **dadurch gekennzeichnet, dass** der oder die Hydrogelkörper (9, 10, 11) in der Mitte der Spirale weiter vorstehen, um eine konvexe Hüllfläche zu bilden.

## Claims

1. An intervertebral disc replacement for the core of an intervertebral disc having a flat spiral (2) made of plastic which can be elongated in a longitudinal direction using auxiliary tools and which automatically coils into its spiral shape in the force-free state, **characterised in that** the spiral (2) has one or more bodies (9, 10, 11) made of a hydrogel on the upper side (18) or lower side (19) along its coiled body (3, 4, 5), said bodies (9, 10, 11) increasing their volumes due to the absorption of water and projecting beyond the upper side (18) or the lower side (19) of the spiral (2).

2. An intervertebral disc replacement in accordance with claim 1, **characterised in that** the spiral (2) consists of a polycarbonate urethane.

3. An intervertebral disc replacement in accordance with claim 1 or claim 2, **characterised in that** the hydrogel body or bodies (9, 10, 11) consist of PVP (polyvinylpyrrolidone), PVA (polyvinyl alcohol), PAN (polyacrylonitrile), PAM (polyacrylamide) or PHEMA (polyhydroxy ethyl metracylate).

4. An intervertebral disc replacement in accordance with any one of claims 1 to 3, **characterised in that** a plurality of individual bodies (11) made of hydrogel are inserted in recesses (12) of the spiral (2) on the upper side (18) or on the lower side (19) along the spiral (2).

5. An intervertebral disc replacement in accordance with any one of claims 1 to 3, **characterised in that** individual bodies (11) made of hydrogel are inserted in openings (13) along the spiral.

6. An intervertebral disc replacement in accordance with any one of claims 1 to 3, **characterised in that** a hydrogel body (9, 10) extends on the upper side or on the lower side along the spiral (2) and is connected thereto.

7. An intervertebral disc replacement in accordance with any one of claims 1 to 3, **characterised in that** the spiral is surrounded by one or more ring-shaped hydrogel bodies (14) over a pre-determined length in the longitudinal direction (20).

8. An intervertebral disc replacement in accordance with any one of claims 1 to 3, **characterised in that** a plurality of spirals (2) made of plastic and a plurality of spiral-shaped hydrogel parts (15) are connected in a sandwich design (16).

9. An intervertebral disc replacement in accordance with any one of claims 1 to 8, **characterised in that** the spiral and the hydrogel parts are connected by an elastic adhesive bond (17).

10. An intervertebral disc replacement in accordance with any one of claims 1 to 8, **characterised in that** the spiral (2) and the hydrogel parts (9, 10, 11) are connected to one another by fusing.

11. An intervertebral disc replacement in accordance with any one of the preceding claims, **characterised in that** turns (3) of the spiral (2) have a step (21) at their upper or lower sides (18, 19) in the longitudinal direction (20) in the swollen state of the hydrogel bodies (9, 10, 11).

12. An intervertebral disc replacement in accordance with claim 4 or claim 5, **characterised in that** the individual bodies (11) are inserted in recesses (12) or openings (13) which have a smaller wall thickness (22) towards the outer side than towards the inner side of the spiral (2) in order to amplify the coiling effect.

13. An intervertebral disc replacement in accordance with any one of the preceding claims, **characterised in that** the hydrogel body or bodies (9, 10, 11) project further at the centre of the spiral in order to form a convex envelope surface.

## Revendications

1. Disque intervertébral de remplacement pour le noyau d'un disque intervertébral, comportant une spirale plate (2) en matière plastique qui peut être étirée longitudinalement avec des outils auxiliaires et qui, à l'état non soumis à des forces, s'enroule automatiquement en sa forme de spirale, **caractérisé en ce que**, le long de son corps enroulé (3, 4, 5), la spirale (2) comporte sur la face supérieure ou inférieure (18, 19) un ou plusieurs corps (9, 10, 11) en un hydrogel, qui agrandissent leur volume par l'absorption d'eau, et qui dépassent de la face supérieure (18) ou de la face inférieure (19) de la spirale (2).

2. Disque intervertébral de remplacement selon la revendication 1, **caractérisé en ce que** la spirale (2) est constituée d'un polycarbonate uréthane.

3. Disque intervertébral de remplacement selon l'une des revendications 1 ou 2, **caractérisé en ce que** le ou les corps (9, 10, 11) en hydrogel sont en PVP (polyvinylpyrrolidone), en PVA (alcool de polyvinyle), en PAN (polyacrylonitrile), en PAM (polyacryamide) ou en PHEMA (polyhydroxyéthylmétracrylate).

4. Disque intervertébral de remplacement selon l'une des revendications 1 à 3, **caractérisé en ce que**, sur la face supérieure (18) ou sur la face inférieure (19), plusieurs corps individuels (11) en hydrogel sont insérés le long de la spirale (2) dans des évidements (12) de la spirale (2).

5. Disque intervertébral de remplacement selon l'une des revendications 1 à 3, **caractérisé en ce que** des corps individuels (11) en hydrogel sont insérés le long de la spirale dans des percées (13).

6. Disque intervertébral de remplacement selon l'une des revendications 1 à 3, **caractérisé en ce qu'**un corps (9, 10) en hydrogel s'étend sur la face supérieure ou sur la face inférieure le long de la spirale (2), et est relié à cette dernière.

7. Disque intervertébral de remplacement selon l'une des revendications 1 à 3, **caractérisé en ce que**, dans la direction longitudinale (20), la spirale est entourée sur une longueur prédéterminée d'un ou de plusieurs corps annulaires (14) en hydrogel.

8. Disque intervertébral de remplacement selon l'une des revendications 1 à 3, **caractérisé en ce que** plusieurs spirales (2) en matière plastique et plusieurs éléments en hydrogel (15) en forme de spirale sont reliés en une construction de type sandwich (16).

9. Disque intervertébral de remplacement selon l'une des revendications 1 à 8, **caractérisé en ce que** des spirales et des éléments en hydrogel sont reliés par une colle élastique (17).

10. Disque intervertébral de remplacement selon l'une des revendications 1 à 8, **caractérisé en ce que** des spirales (2) et des éléments (9, 10 11) en hydrogel sont reliés entre eux par fusion.

11. Disque intervertébral de remplacement selon l'une des revendications précédentes, **caractérisé en ce que**, à l'état gonflé des corps (9, 10, 11) en hydrogel, des spires (3) de la spirale (2) comportent dans la direction longitudinale (20) sur leur face supérieure ou inférieure (18, 19) un gradin (21).

12. Disque intervertébral de remplacement selon la revendication 4 ou 5, **caractérisé en ce que** les corps individuels (11) sont insérés dans des évidements (12) ou dans des percées (13), dont l'épaisseur de paroi (22) est plus faible vers la face extérieure que vers la face intérieure de la spirale (2) afin de renforcer l'effet d'enroulement.

13. Disque intervertébral de remplacement selon l'une des revendications précédentes, **caractérisé en ce que**, au centre de la spirale, le ou les corps (9, 10, 11) en hydrogel dépassent davantage afin de former une surface d'enveloppe convexe.
